# FASCICULE DE BREVET EUROPEEN

(11) **EP 1 148 849 B1**
(45) Date de publication et mention de la délivrance du brevet: **12.12.2007**
(21) Numéro de dépôt: 00901709.6
(22) Date de dépôt: 03.02.2000
(51) Int. Cl.: A61F 2/44

(54) **VIS DE LIAISON REHABITABLE POUR L'ARTHRODESE D'UNE ARTICULATION OSSEUSE, NOTAMMENT POUR LA STABILISATION DE DEUX VERTEBRES**
WIEDEREINWACHSBARE VERBINDUNGSSCHRAUBE ZUR ARTHRODESE EINES KNOCHENGELENKS, INSBESONDERE ZUR STABILISIERUNG ZWEIER WIRBEL
REHABITABLE LINKING SCREW FOR ARTHRODESIS OF A BONE JOINT FOR STABILISING TWO VERTEBRAE

(30) Priorité: 04.02.1999 FR 9901303
(43) Date de publication de la demande: 31.10.2001
(73) Titulaire: Aesculap AG & Co. KG, 78532 Tuttlingen (DE)
(72) Inventeur: AMEIL, Marc, F-51100 Reims (FR); HUPPERT, Jean, F-42580 l'Etrat (FR); MARNAY, Thierry, F-34000 Montpellier (FR)
(74) Mandataire: Eidelsberg, Olivier Nathan
(86) Numéro de dépôt international: PCT/FR2000/000252
(87) Numéro de publication internationale: WO 2000/045752

(56) Documents cités:
- EP-A- 0 637 440
- EP-A- 0 784 967
- WO-A-92/14423
- WO-A-95/25487
- WO-A-98/02117
- WO-A-98/17209
- FR-A- 2 736 538
- FR-A- 2 766 085
- US-A- 5 609 636

## Description

La présente invention est relative à un implant de liaison réhabitable pour la réalisation de l'arthrodèse d'une articulation osseuse, et elle s'applique en particulier, mais de manière non limitative, à un tel implant de liaison utilisé pour la stabilisation de deux vertèbres adjacentes.

Dans cette application particulière, il s'agit de solidariser l'une avec l'autre deux vertèbres adjacentes, par exemple en cas de lésion irrémédiable du disque intervertébral correspondant.

De tels implants de liaison comportent, de manière habituelle, un corps fileté dont la surface périphérique présente des perforations qui sont en communication avec une cavité intérieure. Cette cavité est destinée à recevoir une substance osseuse, formant greffon, dont la finalité est de fusionner avec la matière osseuse vertébrale environnante. On réalise ainsi une arthrodèse, à la fois par voie mécanique du fait que la vis est vissée, sur une partie de son contour, dans les corps vertébraux, et par voie osseuse du fait que le greffon se lie de manière définitive aux deux vertèbres adjacentes.

On connaît déjà par le certificat d'utilité N° 95 08413 une vis de liaison du genre décrit précédemment. Cependant, les deux parties extrêmes comportent des reliefs anti-recul ou crantage destinés à être introduits en force ou par impactage avec deux vertèbres. Cette introduction forcée ou par impactage peut être traumatisante aussi bien pour le patient, si elle est réalisée trop brutalement, que pour le médecin qui peut avoir peur de trop forcer et donc mal fixer l'implant de liaison vertébrale entre les vertèbres.

Le document WO92/14423 au nom de MADHAVAN décrit un implant de liaison muni de fenêtres permettant à une cavité intérieure de déboucher à l'extérieur. Cependant, ces fentes sont très minces et ne permettent pas le passage de substance osseuse à travers elles. Leur fonction est de faciliter la flexion de l'implant suivant l'axe longitudinal pour aider à son insertion, pas d'aider à la fusion osseuse.

Le document FR-A-2.766.085 (CARLIER) prévoit un filetage d'un seul côté de l'implant et celui-ci ne s'étend pas jusqu'au bout extérieur de l'implant. De l'autre côté, il est prévu des moyens de maintien provisoire de l'implant entre les structures osseuses et l'implant. Cependant, il n'est prévu ni décrit nulle part que ces moyens puissent être constitués d'un filetage.

Le document EP-0.784.967 (HOWMEDICA) décrit un implant ayant une partie centrale filetée. Cependant, les parties extrêmes de l'implant ne sont pas filetées extérieurement.

L'invention a pour but de fournir un implant de liaison qui, tout en assurant un excellent ancrage mécanique dans les vertèbres, permet d'augmenter sa capacité de fusion osseuse, et qui en outre est plus facile à introduire dans les vertèbres, notamment sans avoir à effectuer un impactage qui peut s'avérer traumatisant.

A cet effet, l'implant de liaison selon l'invention est défini dans la revendication 1.

Ainsi, avec l'implant selon l'invention, on obtient, d'une part, de pouvoir visser lentement l'implant ou vis dans les vertèbres à relier, ce qui permet d'effectuer une solidarisation de cet implant dans les vertèbres de manière douce et donc non traumatisante, et d'autre part, une excellente capacité de fusion osseuse avec les deux vertèbres adjacentes du fait que la partie centrale de l'implant peut déboucher sur l'extérieur par deux grandes fenêtres.

En outre, lors du vissage lent il est possible que de la matière osseuse soit raclée de la vertèbre et vienne dans la cavité, améliorant encore plus la capacité de fusion osseuse.

Suivant un perfectionnement de l'invention, le corps oblong monobloc est creux. La préparation de l'implant de liaison, et notamment la préparation de la cavité, en est simplifiée.

Suivant un perfectionnement de l'invention, on prévoit que les deux parties extrêmes sont filetées, chaque filetage s'étendant jusqu'au bout extérieur respectif de la partie extrême correspondante. On obtient ainsi une excellente tenue mécanique de l'implant grâce aux deux filetages extrêmes.

De préférence, le corps de la vis est à base de fibres de carbone.

Suivant un mode de réalisation, les deux parties extrêmes filetées peuvent avoir le même diamètre ; en variante, le corps peut présenter une légère conicité, par exemple de l'ordre de 2° à 5°, pour s'amincir dans le sens antéro-postérieur.

Les fenêtres de la partie centrale s'étendent axialement sur au moins toute la longueur de celle-ci.

En section droite transversale, la partie centrale de la vis est inscrite dans un cercle dont le diamètre est soit légèrement supérieur au diamètre du fond de filet et inférieur au diamètre du sommet de filet des parties extrêmes, soit légèrement inférieur au diamètre du fond de filet de la ou des parties extrêmes.

L'une des deux parties extrêmes peut présenter un alésage, par exemple taraudé, pour la réception d'un élément qui appartient à un équipement ancillaire et/ou à un système de liaison intervertébrale.

La partie centrale de la vis présente avantageusement deux méplats extérieurs axiaux parallèles, de préférence sur toute sa longueur et parallèlement à la direction suivant laquelle la cavité centrale débouche sur l'extérieur.

Ces méplats, lors de la mise en place de l'implant, viennent en contact "à plat" avec les plateaux vertébraux. Ceci garantit une meilleure stabilité en comparaison des contacts en "arête" des implants de l'art antérieur.

De préférence également, le filet de chacune des deux parties extrêmes est ininterrompu.

De préférence, les filetages des deux parties extrêmes présentent le même pas, et la distance axiale séparant deux spires respectives de ces filetages est égale à un multiple de ce pas.

Enfin, et spécialement dans le cas où le corps de la vis est à base de fibres de carbone, le filet de chaque partie extrême présente une section droite axiale comportant un sommet étroit et plat, un fond large et plat, et des flancs courbes, par exemple concaves et en arc de cercle.

Suivant un mode de réalisation avantageux, en section transversale, les méplats ont un profil oblong, délimité par deux segments de droites rectilignes transversales parallèles de dimension différentes, reliées à leurs extrémités par des segments circulaires.

Suivant un mode de réalisation avantageux, les implants ont, en section transversale, un profil oblong, en forme de demi-octogone.

On comprendra bien l'invention à la lecture du complément de description qui va suivre et en référence aux dessins annexés qui font partie de la description et dans lesquels :
Fig. 1 est une vue en élévation d'une vis de liaison établie selon un premier mode de réalisation de l'invention ;
Fig. 2 est une vue en plan de la vis de la Fig. 1 ;
Fig. 3 est une vue en coupe transversale de la vis de la Fig. 1 suivant la ligne III-III ;
Fig. 4 est une vue en bout de la vis de la Fig. 1 ;
Fig. 5 est une vue en coupe axiale de la vis de la Fig. 2 suivant la ligne V-V ;
Fig. 6 montre en coupe axiale et à plus grande échelle la forme du filet des parties extrêmes de la vis ;
Fig. 7 est une vue en élévation d'une vis de liaison établie suivant un second mode de réalisation de l'invention ;
Fig. 8 est une vue en plan de la vis de la Fig. 7 ;
Fig. 9 est une vue en coupe transversale de la vis de la Fig. 7 suivant la ligne IX-IX ;
Fig. 10 est une vue en bout de la vis de la Fig. 7 ;
Fig. 11 est une vue en coupe axiale de la vis de la Fig. 8 suivant la ligne XI-XI ;
Fig. 12 est une vue en élévation d'une vis de liaison établie selon un troisième mode de réalisation, la vis étant de forme conique ;
Fig. 13 est une vue en plan de la vis de la Fig. 12 ;
Fig. 14 est une vue en coupe transversale de la vis de la Fig. 12 suivant la ligne IV-IV ;
Fig. 15 est une vue en bout de la vis de la Fig. 12 ;
Fig. 16 est une vue en élévation d'une vis de liaison établie selon un quatrième mode de réalisation ;
Fig. 17 est une vue en plan de la vis de la Fig. 16 ;
Fig. 18 est une vue en coupe transversale de la vis de la Fig. 16 suivant la ligne I-I ;
Fig. 19 est une vue en bout de la vis de la Fig. 16 ;
Fig. 20 est une vue en élévation d'un cinquième mode de réalisation de la vis de liaison suivant l'invention,
Fig. 21 est une vue en plan de la vis de la figure 20,
Fig. 22 est une vue en coupe transversale de la vis de la figure 20, suivant la ligne I'-I',
Fig. 23 est une vue en coupe de la figure 20.

On a représenté sur les dessins une vis de liaison établie suivant deux modes de réalisation préférés de l'invention.

On décrira tout d'abord les caractéristiques communes à ces deux modes de réalisation.

Cette vis de liaison comporte un corps allongé monobloc 1, par exemple à base de fibres de carbone, qui est formé, d'une part, par deux parties extrêmes 2 et 3 et, d'autre part, par une partie centrale 4. Le corps 1 présente par exemple une longueur de l'ordre de 26 mm.

Les deux parties extrêmes 2, 3 sont filetées extérieurement, tandis que la partie centrale 4 est lisse. Les filetages 5 et 6 respectivement des parties extrêmes 2, 3 s'étendent sur seulement quelques spires, par exemple deux spires.

La partie centrale lisse 4 présente, sur une partie au moins de sa longueur axiale, une cavité 7 qui est destinée à recevoir, de préférence sur tout son volume, une substance osseuse de fusion vertébrale. Cette cavité débouche à l'extérieur par deux fenêtres 8 et 9 qui sont diamétralement opposées pour être en regard, lorsque la vis est en place, des deux vertèbres à stabiliser.

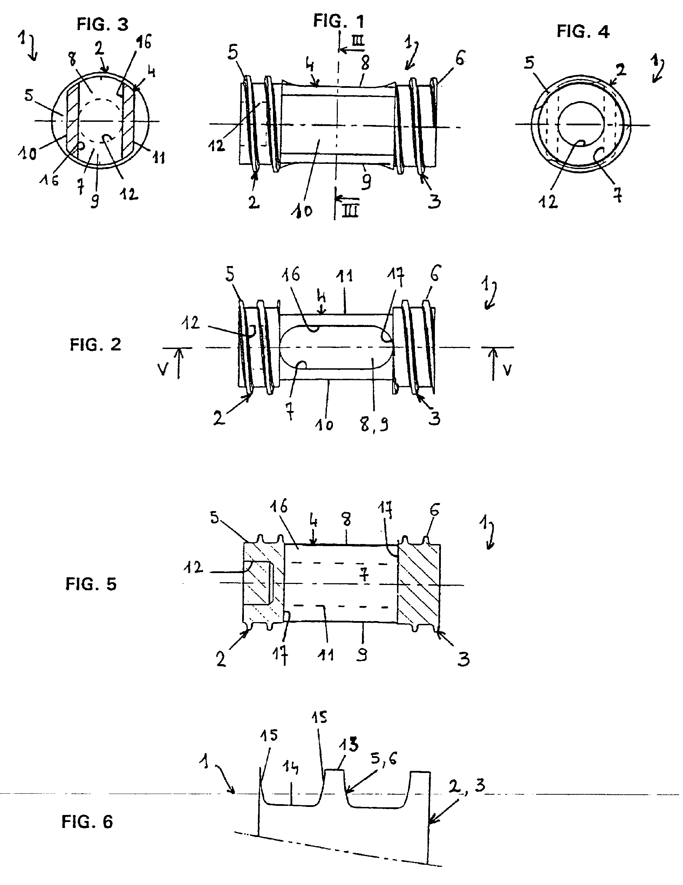

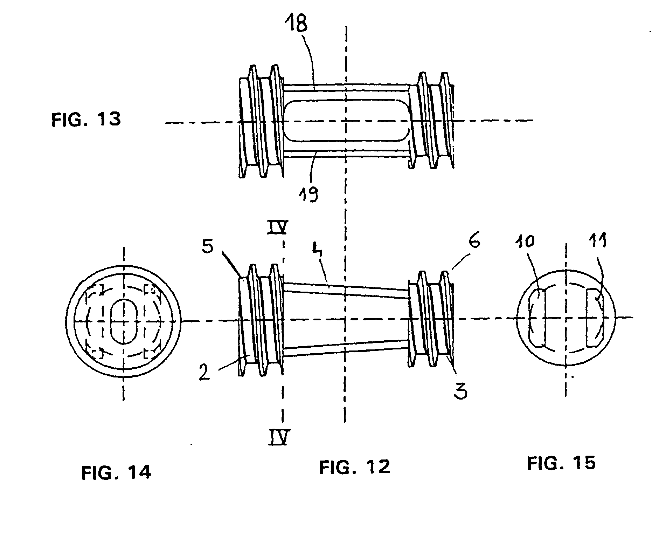

Aux figures 1 à 11, la vis présente une forme générale cylindrique, c'est-à-dire que les deux parties extrêmes filetées 2 et 3 ont le même diamètre ; en variante, comme représenté notamment à la figure 12, il est possible de conférer à la vis une légère conicité, par exemple de l'ordre de 2 à 5°, pour qu'elle s'amincisse dans le sens antéro-postérieur, de manière à restituer, le cas échéant, la lordose naturelle du rachis. Dans ce cas, les deux filetages sont de diamètres différents mais constants (seule la partie centrale est conique).

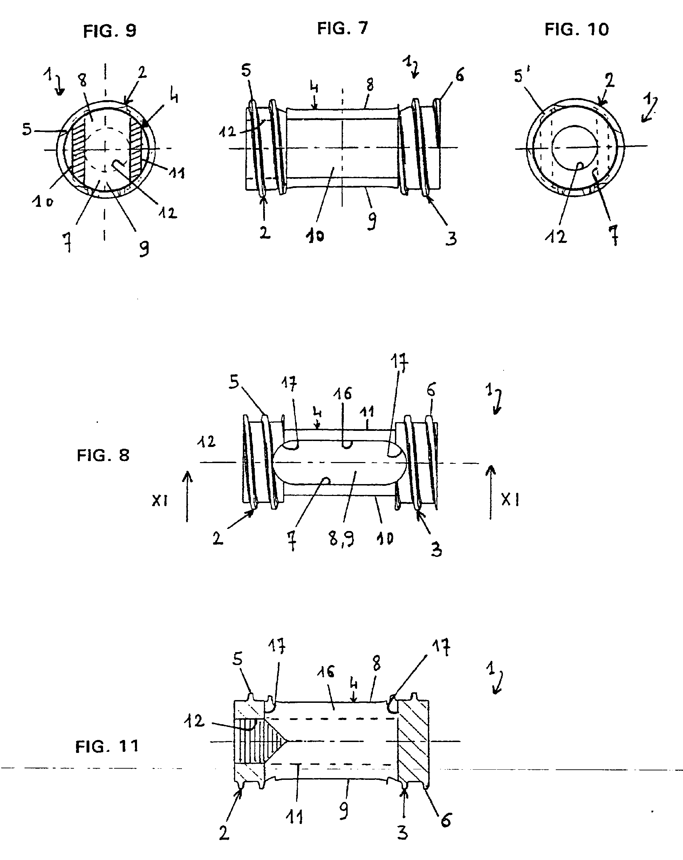

La cavité 7 est définie, dans les exemples représentés, par deux parois axiales parallèles 16 espacées, qui se raccordent à chaque extrémité à un demi-cylindre 17. Dans ces exemples, la cavité 7 et les fenêtres 8 et 9 ont donc la même largeur transversale et la même longueur axiale. En variante, on peut donner à la paroi de la cavité une forme convexe ou concave sur une partie au moins de son contour. L'épaisseur de la cavité 7 dans le sens transversal (Figs. 2 et 8), est sensiblement égale à la moitié de l'épaisseur totale de la partie centrale de la vis.

La cavité 7 est située entre deux méplats extérieurs axiaux parallèles 10 et 11 qui, de préférence, s'étendent sur toute la longueur de la partie centrale 4 en étant parallèles à la direction suivant laquelle la cavité 7 débouche sur l'extérieur par les fenêtres 8 et 9. Les méplats 10 et 11 s'étendent donc dans une direction antéro-postérieure. De préférence, ils n'interfèrent pas avec les filetages 5 et 6.

L'une des deux parties extrêmes filetées 2 et 3, c'est-à-dire celle de ces extrémités qui pénètre la dernière dans l'espace intervertébral, présente un alésage 12, par exemple taraudé, pour la réception d'un élément, non représenté, qui appartient à un équipement ancillaire de mise en place de la vis et/ou à un système de liaison intervertébrale, comportant par exemple au moins une plaque ou au moins une barre. L'alésage 12 peut être borgne (Figs. 1-5) ou déboucher dans la cavité 7 (Figs. 7-11).

On a représenté en coupe axiale sur la Fig. 6, et à plus grande échelle, un profil préféré pour chacun des filetages 5 et 6. Dans l'exemple considéré d'une vis ayant une longueur d'environ 26 mm, chaque partie extrême 2, 3 présente une longueur d'environ 5,5 mm, de sorte que la partie centrale 4 a une longueur d'environ 15 mm. Le pas de chaque filetage 5, 6 est par exemple de 2,75 mm.

Le profil du filet comporte un sommet étroit et plat 13, par exemple de 0,5 mm, un fond large et plat 14 de 1,4 à 1,9 mm, et des flancs courbes 15, par exemple concaves et en arc de cercle, d'un rayon par exemple de 3 mm.

La profondeur du filet peut varier, par exemple, de 1 mm à 1,6 mm.

Dans les deux modes de réalisation représentés, la partie centrale 4 est inscrite, en section droite transversale (Figs. 3 et 9), dans un cercle, c'est-à-dire que la partie centrale 4, entré la cavité 7 et les méplats 10 et 11, présente une surface extérieure cylindrique ou légèrement conique.

Dans le mode de réalisation des Figs. 1 à 5, ce cercle, ou le cas échéant la grande base du tronc de cône, présente un diamètre qui est légèrement supérieur au diamètre du fond de filet 14, tout en étant inférieur au diamètre du sommet de filet 13 des parties extrêmes. Cela permet, lors de la mise en place de la vis, d'obtenir non seulement un ancrage de la vis dans les deux vertèbres par les filetages 5 et 6, mais également un serrage de la partie centrale 4.

Au contraire, comme c'est le cas dans le mode de réalisation des Figs. 7 à 11, si on ne souhaite pas réaliser un tel serrage de la partie centrale 4, ce cercle, ou le cas échéant la grande base du tronc de cône, présente un diamètre qui est inférieur au diamètre du fond de filet 14 des parties extrêmes 2 et 3.

Une autre différence entre les deux modes de réalisation représentés réside en ce que, sur les Figs. 1 à 5, chacun des filets 5 et 6 est ininterrompu, c'est-à-dire que les deux fenêtres 8 et 9 n'interfèrent pas avec lui tandis que, sur les Figs. 7 à 11, les fenêtres 8 et 9 s'étendent axialement sur une longueur supérieure à celle de la partie centrale 4, de sorte qu'elles interfèrent au moins une fois avec la spire la plus intérieure des filetages, comme on le voit au mieux sur la Fig. 8.

Ainsi, les fenêtres peuvent s'étendre soit au moins sur une partie de la longueur de la partie centrale, soit, de préférence, sur au moins toute la longueur de celle-ci.

Bien entendu, dans les deux modes de réalisation ci-dessus, les filetages 5, 6 des deux parties extrêmes 2, 3 présentent le même pas, et la distance axiale séparant deux spires respectives de ces filetages est égale à un multiple de ce pas, de telle manière que le filetage de la partie extrême 2, qui pénètre en dernier dans l'espace intervertébral, vienne en congruence avec le taraudage ménagé pour le vissage initial de la partie extrême 3.

Pour la mise en place de la vis selon l'invention, éventuellement après ablation du disque intervertébral, on réalise dans l'espace intervertébral un alésage, auquel on fait de préférence comporter un taraudage ou une ébauche de taraudage.

Avant cette mise en place de la vis, la cavité centrale 7 est remplie de substance osseuse qui ultérieurement, à travers les deux fenêtres 8 et 9, va venir fusionner avec la matière osseuse des plateaux vertébraux adjacents.

Un troisième mode de réalisation est représenté aux figures 12 à 15.

Dans ce mode de réalisation, la partie centrale 4 est en forme de biseau, en s'amincissant dans le sens antéro-postérieur. Les deux parties extrêmes 2, 3 sont filetées extérieurement, les deux filetages 5, 6 étant de diamètres différents, mais constants.

La vue en plan (figure 13) de ce troisième mode de réalisation est identique à celle du premier mode de réalisation (figure 2), à l'exception de la présence des arêtes vives 18 et 19, qui correspondent au fait que le profil des deux méplats 10 et 11, en coupe transversale est à angle vif, et non plus arrondi en s'inscrivant dans un cercle. Ils ont un profil, en section transversale (figures 14 et 15) sous la forme d'un demi octogone oblong dans le sens transversal à la direction antéro-postérieure (gauche-droite aux figures).

Les deux méplats 10 et 11, compte tenu de la forme en biseau de la partie centrale 4, n'ont pas, en coupe transversale (au niveau de la partie extrême 2 (figure 14) les mêmes dimensions qu'au niveau de la partie extrême 3 (figure 13).

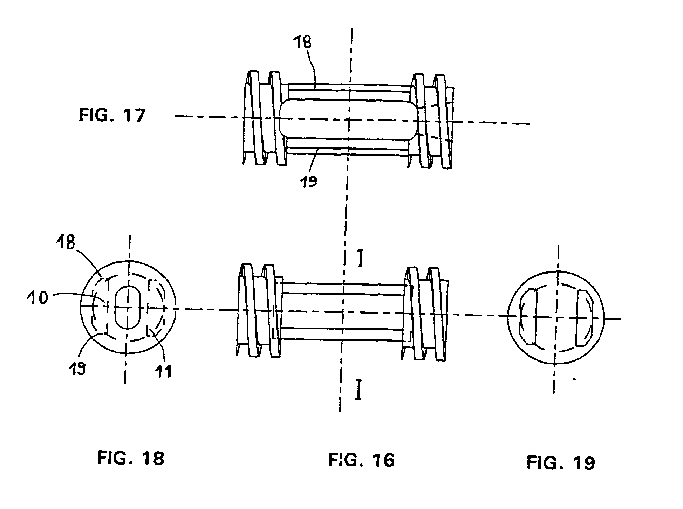

Cette forme du profil transversal des méplats peut être prévue également dans le cas où la partie centrale n'est pas en forme de biseau, comme cela est représenté au quatrième mode de réalisation des figures 16 à 19.

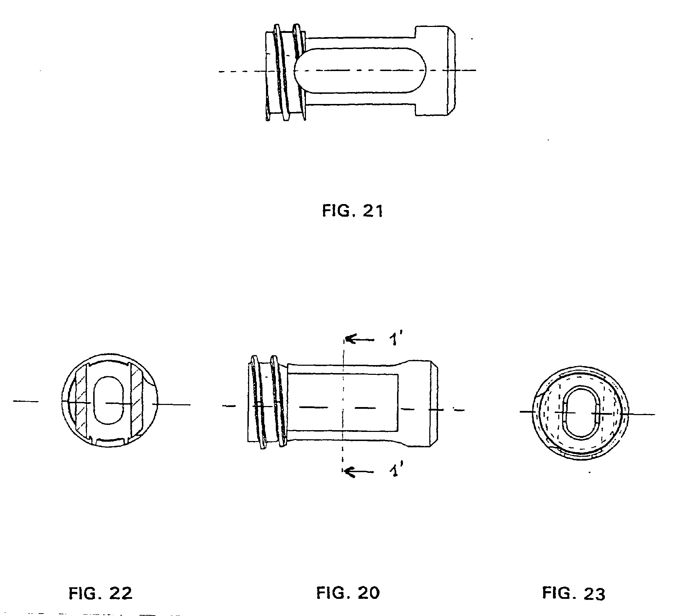

Les figures 20 à 23 représentent un cinquième mode de réalisation dans lequel une seule partie extrême est filetée. Cela permet d'obtenir une vis de liaison moins coûteuse et plus facile à fabriquer,

Bien entendu, l'invention n'est pas limitée aux modes de réalisation qui ont été décrits ; on pourrait au contraire concevoir diverses variantes sans sortir pour autant du cadre des revendications, notamment combiner les modes de réalisation décrits.

## Revendications

1. Implant de liaison réhabitable pour l'arthrodèse d'une articulation osseuse, notamment pour la stabilisation de deux vertèbres adjacentes, constitué par un corps oblong monobloc (1) formé de deux parties extrêmes (2, 3) et d'une partie centrale lisse (4), munie sur une partie au moins de sa longueur d'une cavité (7) qui est destinée à recevoir une substance osseuse de fusion vertébrale qui débouche à l'extérieur par deux fenêtres (8, 9) diamétralement opposées, en regard des deux vertèbres à stabiliser, et de dimensions suffisantes pour y permettre le passage de substance osseuse de fusion, **caractérisé en ce que** au moins l'une (2) des parties extrêmes (2, 3) est filetée extérieurement, jusqu'au bout extérieur de celle-ci et **en ce que** en section transversale, les fenêtres de la partie centrale ont une longueur axiale et une longueur transversale identiques à celles de la cavité et les fenêtres sont délimitées par deux segments de droites rectilignes transversales parallèles, reliées à leurs extrémités par des segments circulaires, et les fenêtres de la partie centrale s'étendent axialement sur au moins toute la longueur de celle-ci.

2. Implant de liaison suivant la revendication 1, **caractérisé en ce que** le corps oblong monobloc (1) est creux.

3. Implant de liaison suivant la revendication 1 ou 2, **caractérisé en ce que** les deux parties (2, 3) extrêmes sont filetées extérieurement, et ce jusqu'à leur bout extérieur respectif.

4. Implant de liaison selon la revendication 1, 2 ou 3, **caractérisé en ce que** le corps est à base de fibres de carbone.

5. Implant de liaison selon l'une des revendications 1 à 4 , **caractérisé en ce que** le corps présente une légère conicité, par exemple de l'ordre de 2° à 5°, pour s'amincir dans le sens antéro-postérieur.

6. Implant de liaison selon l'une des revendications 1 à 5, **caractérisé en ce que** les deux parties extrêmes ont le même diamètre.

7. Implant de liaison selon l'une des revendications 1 à 6, **caractérisé en ce que**, en section droite transversale, la partie centrale est inscrite dans un cercle dont le diamètre est légèrement supérieur au diamètre du fond de filet (14) et inférieur au diamètre du sommet de filet (13) de la ou des parties extrêmes filetées.

8. Implant de liaison selon l'une des revendications 1 à 6, **caractérisé en ce que**, en section droite transversale, la partie centrale est inscrite dans un cercle dont le diamètre est légèrement inférieur au diamètre du fond de filet (14) de la ou des parties extrêmes filetées.

9. Implant de liaison selon l'une des revendications 1 à 8, **caractérisée en ce que** l'une des deux parties extrêmes présente un alésage (12), par exemple taraudé, pour la réception d'un élément qui appartient à un équipement ancillaire et/ou à un système de liaison intervertébrale, cet alésage étant borgne ou débouchant dans la cavité de la partie centrale.

10. Implant de liaison selon l'une des revendications 1 à 9, **caractérisé en ce que** la partie centrale présente deux méplats extérieurs axiaux parallèles (10, 11), de préférence sur toute sa longueur et parallèlement à la direction suivant laquelle la cavité centrale débouche sur l'extérieur, ces méplats étant situés de part et d'autre de la cavité de la partie centrale.

11. Implant de liaison selon l'une des revendications 1 à 10, **caractérisé en ce que** le filet (5, 6) de chacune de la ou des parties extrêmes est ininterrompu.

12. Implant de liaison selon l'une des revendications 1 à 11, **caractérisée en ce que**, sur chaque partie extrême filetée, le filet (5, 6) présente une section droite axiale comportant un sommet étroit et plat (13), un fond large et plat (14), et des flancs courbes (15), par exemple concaves (15), et en arc de cercle.

13. Implant de liaison selon l'une des revendications 2 à 12, **caractérisé en ce que** les filetages (5, 6) des deux parties extrêmes présentent le même pas, et la distance axiale séparant deux spires respectives de ces filetages est égale à un multiple de ce pas.

14. Implant de liaison selon l'une des revendications 10 à 11, **caractérisée en ce qu'**en section transversale, les méplats (10, 11) ont un profil oblong en forme de demi-octogone.

15. Implant de liaison selon l'une des revendications 10 à 13, **caractérisé en ce qu'**en section transversale, les implants (10, 11) ont un profil oblong, délimité par deux segments de droites rectilignes transversales parallèles de dimensions différentes, reliées à leurs extrémités par des segments circulaires.

## Claims

1. Rehabitable linking implant for arthrodesis of an osseous joint, notably for stabilising two adjacent vertebrae, consisting of an oblong main section cast in one piece (1) formed by two end sections (2,3) and one smooth central section (4), provided, on at least one section of its length, with a cavity (7) which is to accommodate an osseous vertebral fusion substance which flows to the outside through two windows (8,9) which are diametrically opposite the two vertebrae to be stabilised and large enough to allow the osseous fusion substance to pass through, whereby at least one (2) of the end sections (2,3) is threaded externally, as far as the outer end of the latter and whereby, in transverse cross-section, the windows of the central section are identical in axial length and transverse length to those of the cavity and the windows are defined by two segments of parallel transverse straight lines linked together at their ends by circular segments, and the windows of the central section extend axially along the entire length of the latter.

2. Linking implant according to claim 1, whereby the oblong main section cast in one piece (1) is hollow.

3. Linking implant according to claim 1 or 2, whereby the two end sections (2,3) are threaded externally as far as their respective outer ends.

4. Linking implant according to claim 1, 2 or 3, whereby the substance has a carbon fibre base.

5. Linking implant according to one of claims 1 to 4, whereby the substance tapers slightly, for example in the region of 2° to 5°, getting thinner in the anteroposterior direction.

6. Linking implant according to one of claims 1 to 5, whereby the two end sections have the same diameter.

7. Linking implant according to one of claims 1 to 6, whereby the central section, as a straight cross-section, is inscribed in a circle, the diameter of which is slightly larger than the diameter of the thread root (14) and smaller than the diameter of the thread crest (13) of the threaded end section(s).

8. Linking implant according to one of claims 1 to 6, whereby the central section, as a straight cross-section, is inscribed in a circle, the diameter of which is slightly smaller than the diameter of the thread root (14) of the threaded end section(s).

9. Linking implant according to one of claims 1 to 8, whereby one of the two end sections has a hole (12), threaded for example, to accommodate an element belonging to an item of ancillary equipment and/or to a system of intervertebral joints, this hole being blind or opening onto the cavity of the central section.

10. Linking implant according to one of claims 1 to 9, whereby the central section has two parallel axial external planes (10,11), preferably over its entire length and parallel to the direction, according to which the central section opens onto the outside, these planes being located on either side of the cavity of the central section.

11. Linking implant according to one of claims 1 to 10, whereby the thread (5,6) of each of the end section(s) is continuous.

12. Linking implant according to one of claims 1 to 11, whereby over each threaded end section the thread (5,6) has an axial cross-section comprising a flat, narrow crest (13), a flat, wide root (14) and curved sides (15), concave for example (15), as an arc of a circle.

13. Linking implant according to one of claims 2 to 12, whereby the threads (5,6) of the two ends sections have the same thread and the axial distance separating two respective turns of these threads is equal to a multiple of this thread.

14. Linking implant according to one of claims 10 to 11, whereby the planes (10,11) in cross-section have an oblong profile in the shape of a half-octagon.

15. Linking implant according to one of claims 10 to 13, whereby the implants (10,11) in cross-section have an oblong profile defined by two segments of parallel transverse rectilinear straight lines of different sizes linked together at their ends by circular segments.

## Patentansprüche

1. Wiedereinwachsfähiges Verbindungsimplantat für die Arthrodese eines Knochengelenks, insbesondere für die Stabilisierung zweier benachbarter Wirbel, das aus einem länglichen einstückigen Körper (1) besteht, der aus zwei Endabschnitten (2,3) und einem glatten, zentralen Abschnitt (4) gebildet und zumindest auf einem Teil seiner Länge mit einer Ausnehmung (7) versehen ist, die dazu vorgesehen ist, eine vertebrale Knochenschmelzsubstanz aufzunehmen, die über zwei diametral entgegengesetzte Fenster (8,9), welche den zu stabilisierenden Wirbeln zugewandt sind und ausreichende Dimensionen aufweisen, um den Durchgang der Knochenschmelzsubstanz zu ermöglichen, nach außen mündet, **dadurch gekennzeichnet, dass** mindestens einer (2) der Endabschnitte (2,3) mit einem Außengewinde bis zu seinem äußeren Ende versehen ist, und dass im Querschnitt die Fenster des zentralen Abschnitts eine axiale Länge und eine transversale Länge aufweisen, die zu denjenigen der Ausnehmung identisch sind, und die Fenster von zwei transversalen parallelen Geradensegmenten begrenzt sind, die an ihren Enden durch kreisförmige Segmente verbunden sind, und die Fenster des zentralen Abschnitts sich axial auf mindestens der gesamten Länge derselben erstrecken.

2. Verbindungsimplantat nach Anspruch 1, **dadurch gekennzeichnet, dass** der längliche einstückige Körper (1) hohl ist.

3. Verbindungsimplantat nach Anspruch 1 oder 2, **dadurch gekennzeichnet, dass** die beiden Endabschnitte (2,3) bis zu ihrem jeweiligen äußeren Ende mit Außengewinden versehen sind.

4. Verbindungsimplantat nach Anspruch 1, 2 oder 3, **dadurch gekennzeichnet, dass** der Körper auf Kohlenstofffasern basiert.

5. Verbindungsimplantat nach einem der Ansprüche 1 bis 4, **dadurch gekennzeichnet, dass** der Körper eine leichte Konizität aufweist, beispielsweise in der Größenordnung von 2° bis 5°, um sich in der antero-posterioren Richtung zu verdünnen.

6. Verbindungsimplantat nach einem der Ansprüche 1 bis 5, **dadurch gekennzeichnet, dass** die beiden äußeren Abschnitte den gleichen Durchmesser aufweisen.

7. Verbindungsimplantat nach einem der Ansprüche 1 bis 6, **dadurch gekennzeichnet, dass** im geraden Querschnitt der zentrale Abschnitt in einen Kreis eingeschrieben ist, dessen Durchmesser geringfügig größer ist als der Durchmesser des Gewindebodens (14) und kleiner ist als der Durchmesser des Gewindeendes (13) des/der mit Gewinde versehenen Endabschnitts/Endabschnitte.

8. Verbindungsimplantat nach einem der Ansprüche 1 bis 6, **dadurch gekennzeichnet, dass** im geraden Querschnitt der zentrale Abschnitt in einen Kreis eingeschrieben ist, dessen Durchmesser geringfügig kleiner ist als der Durchmesser des Gewindebodens (14) des/der mit Gewinde versehenen Endabschnitts/Endabschnitte.

9. Verbindungsimplantat nach einem der Ansprüche 1 bis 8, **dadurch gekennzeichnet, dass** einer seiner Endabschnitte eine beispielsweise mit Gewindebohrer eingebrachte Bohrung (12) für die Aufnahme eines Elements aufweist, das zu einer Hilfseinrichtung und/oder zu einem Zwischenwirbel-Verbindungssystem gehört, wobei diese Bohrung eine Blindbohrung ist oder in die Ausnehmung des zentralen Abschnitts mündet.

10. Verbindungsimplantat nach einem der Ansprüche 1 bis 9, **dadurch gekennzeichnet, dass** der zentrale Abschnitt zwei axiale parallele äußere Abflachungen (10,11) vorzugsweise auf seiner gesamten Länge und parallel zu der Richtung aufweist, in der die zentrale Ausnehmung nach außen mündet, wobei diese Abflachungen auf beiden Seiten der Ausnehmung des zentralen Abschnitts gelegen sind.

11. Verbindungsimplantat nach einem der Ansprüche 1 bis 10, **dadurch gekennzeichnet, dass** das Gewinde (5,6) jedes Endabschnitts/ jedes der Endabschnitte ununterbrochen ist.

12. Verbindungsimplantat nach einem der Ansprüche 1 bis 11, **dadurch gekennzeichnet, dass** an jedem mit Gewinde versehenen Endabschnitt das Gewinde (5,6) einen axialen geraden Querschnitt aufweist, der einen schmalen und flachen Scheitel (13), einen breiten und flachen Boden (14) sowie gekrümmte Flanken (15), beispielsweise konkave Flanken (15) in Kreisbogenform, aufweist.

13. Verbindungsimplantat nach einem der Ansprüche 2 bis 12, **dadurch gekennzeichnet, dass** die Gewinde (5,6) der beiden Endabschnitte die gleiche Teilung aufweisen, und der axiale Abstand, welcher zwei jeweilige Gewindegänge dieser Gewinde voneinander trennt, gleich einem Vielfachen dieser Teilung ist.

14. Verbindungsimplantat nach einem der Ansprüche 10 bis 11, **dadurch gekennzeichnet, dass** in einem geraden Querschnitt die Abflachungen (10,11) ein längliches Profil in Form eines halben Achtecks haben.

15. Verbindungsimplantat nach einem der Ansprüche 10 bis 13, **dadurch gekennzeichnet, dass** im Querschnitt die Abflachungen (10,11) ein längliches Profil aufweisen, das von zwei geraden transversalen parallelen Segmenten unterschiedlicher Dimensionen begrenzt ist, die an ihren Enden durch kreisförmige Segmente verbunden sind.
